# EUROPÄISCHE PATENTSCHRIFT

(11) **EP 2 613 726 B1**
(45) Veröffentlichungstag und Bekanntmachung des Hinweises auf die Patenterteilung: **05.10.2016**
(21) Anmeldenummer: 11758384.9
(22) Anmeldetag: 31.08.2011
(51) Int. Cl.: A61B 17/00

(54) **CHIRURGISCHES INSTRUMENT**
SURGICAL INSTRUMENT
INSTRUMENT CHIRURGICAL

(30) Priorität: 10.09.2010 DE 102010044982
(43) Veröffentlichungstag der Anmeldung: 17.07.2013
(73) Patentinhaber: Dan Med&Repair Dannoritzer Medizintechnik GmbH & Co. KG, 78532 Tuttlingen (DE)
(72) Erfinder: DANNORITZER, Axel, 78532 Tuttlingen (DE)
(74) Vertreter: Daub, Thomas
(86) Internationale Anmeldenummer: PCT/EP2011/004382
(87) Internationale Veröffentlichungsnummer: WO 2012/031712

(56) Entgegenhaltungen:
- WO-A1-97/24072
- DE-A1- 4 316 768
- DE-U1-202004 015 642
- US-A- 5 618 308
- US-A1- 2004 230 221

## Beschreibung

### Stand der Technik

Die Erfindung geht insbesondere aus von einem chirurgischen Instrument nach dem Oberbegriff des Anspruchs 1.

Aus der US 2004/230221 ist ein minimal-invasives chirurgisches Instrument mit einer Arbeitseinheit und einer Bedieneinheit bekannt. Das chirurgische Instrument weist eine Verbindungsvorrichtung auf, die die Arbeitseinheit und die Bedieneinheit zur Reinigung und/oder Desinfizierung trennbar miteinander verbindet.

Aus der US 5,618,308 A ist ein minimal-invasives chirurgisches Instrument mit einer Arbeitseinheit und einer Bedieneinheit bekannt. Die Bedieneinheit umfasst zwei Bedienelemente, die über einen Knopf zur Reinigung und/oder Desinfizierung voneinander trennbar miteinander verbunden sind.

### Vorteile der Erfindung

Die Erfindung geht aus von einem chirurgischen Instrument, insbesondere einem minimal-invasiven chirurgischen Instrument, mit einer Arbeitseinheit und einer Bedieneinheit, die zwei zur Reinigung und/oder Desinfizierung voneinander trennbare Bedienelemente aufweist und einer Verbindungsvorrichtung, die dazu vorgesehen ist, die Arbeitseinheit und die Bedieneinheit zur Reinigung und/oder Desinfizierung trennbar miteinander zu verbinden, wobei die Arbeitseinheit (12) zumindest einen Bewegungsanschlag (26) aufweist.

Es wird vorgeschlagen, dass der Bewegungsanschlag (26) dazu vorgesehen ist, die Bedienelemente (16, 18) unverlierbar zueinander anzuordnen, wobei der Bewegungsanschlag (26) in einem betriebsbereiten Zustand verhindert, dass die Bedienelemente (16, 18) so zueinander ausrichtbar sind, dass eine formschlüssige Verbindung zwischen den beiden Bedienelementen (16, 18) lösbar ist. Unter einem "chirurgischen Instrument" soll insbesondere ein Instrument verstanden werden, das für dem Fachmann als sinnvoll erscheinende Operationen und vorteilhaft eine minimal-invasive Operation vorgesehen ist. Für minimal-invasive Operationen weist das Instrument zwischen einem Arbeitsmittel der Arbeitseinheit und der Bedieneinheit einen Schaftbereich auf, der einen möglichst geringen maximalen Durchmesser senkrecht zu einer Haupterstreckungsrichtung aufweist, und zwar vorteilhaft weniger als 8 mm, besonders vorteilhaft weniger als 5 mm. Bevorzugt weist der Schaftbereich über eine gesamte Länge in Haupterstreckungsrichtung diesen geringen Durchmesser auf. Vorzugsweise ist der Schaftbereich zwischen einem Arbeitsmittel und einer Bedieneinheit in einem betriebsbereiten Zustand mehr als 50 mm, vorteilhaft mehr als 70 mm, besonders vorteilhaft mehr als 100 mm lang. Unter "minimal-invasiv" soll insbesondere für eine Laparoskopie, eine Thorakoskopie, endoskopische Operationen, andere dem Fachmann als sinnvoll erscheinend minimal-invasive Operationen und vorteilhaft eine Anthroposophie, geeignet verstanden werden. Das chirurgische Instrument kann verschiedene, dem Fachmann als sinnvoll erscheinende Funktionen, wie insbesondere eine Funktion einer Schere und/oder vorteilhaft eine Funktion einer Zange erfüllen. Dabei sind dem Fachmann unterschiedliche Zangen- und/oder Scherenformen bekannt. Unter einer "Bedieneinheit" soll insbesondere eine Einheit verstanden werden, die dazu vorgesehen ist, eine von einem Bediener ausgehende mechanische Kraft aufzunehmen, wobei der Schaftbereich die Kraft an das Arbeitsmittel weiterleitet. Die Bedieneinheit weist vorzugsweise zwei zueinander schwenkbar gelagerte Bedienelemente auf, die vorzugsweise dazu vorgesehen sind, von einem Bediener gegriffen zu werden. Vorzugsweise ist die Bedieneinheit scherengriffartig und/oder zangengriffartig ausgebildet. Unter "vorgesehen" soll insbesondere speziell ausgestattet und/oder ausgelegt verstanden werden. Insbesondere soll unter einer "Arbeitseinheit" eine Einheit verstanden werden, die mit der Bedieneinheit verbindbar ist und die das Arbeitsmittel aufweist. Vorzugsweise ist die Arbeitseinheit dazu vorgesehen, bei einem insbesondere minimal-invasiven chirurgischen Eingriff zumindest teilweise durch eine Hautöffnung in eine Operationsstelle eingeführt zu werden. Insbesondere soll unter dem Begriff "Bedienelement" ein Element verstanden werden, das zumindest einen Griffbereich aufweist, der dazu vorgesehen ist, von einem Bediener bei einem Arbeitsvorgang betätigt zu werden. Unter der Wendung "zur Reinigung und/oder Desinfizierung voneinander trennbar" soll insbesondere verstanden werden, dass zumindest die Arbeitseinheit und die Bedienelemente zur Reinigung und/oder Desinfizierung jeweils räumlich voneinander getrennt anordenbar sind. Unter einer "Verbindungsvorrichtung" soll insbesondere eine Vorrichtung verstanden werden, die dazu vorgesehen ist, die Arbeitseinheit und die Bedieneinheit kraftschlüssig und/oder vorteilhaft formschlüssig fest zu verbinden. Vorzugsweise verrastet die Verbindungsvorrichtung die Arbeitseinheit und die Bedieneinheit miteinander. Insbesondere soll unter der Wendung "miteinander verbinden" verstanden werden, dass die Verbindungsvorrichtung eine Bewegung der gesamten Arbeitseinheit relativ zu der gesamten Bedieneinheit verhindert. Während einer Benutzung kann eine ungewollte Trennung vermieden und dadurch eine besonders komfortable Benutzung erreicht werden. Unter einem "Bewegungsanschlag" soll insbesondere eine Struktur und/oder Anordnung verstanden werden, die dazu vorgesehen ist, zumindest eine Bewegung durch eine Gegenkraft zu verhindern. Somit ist der Bewegungsanschlag der Arbeitseinheit ein Teil der formschlüssigen Verbindung zwischen den beiden Bedienelementen.

Durch die erfindungsgemäße Ausgestaltung des chirurgischen Instruments kann konstruktiv einfach eine besonders vorteilhafte Reinigbarkeit und/oder Desinfizierbarkeit erreicht werden. Des Weiteren kann ein besonders flexibles chirurgisches Instrument bereitgestellt werden, bei dem die Bedieneinheit mit verschiedenen Arbeitseinheiten kombinierbar ist.

In einer weiteren Ausgestaltung wird vorgeschlagen, dass die Verbindungsvorrichtung dazu vorgesehen ist, die Arbeitseinheit und die Bedieneinheit werkzeuglos trennbar zu verbinden, wodurch das chirurgische Instrument besonders komfortabel und schnell zur Reinigung und/oder Desinfizierung und zum Austauschen der Arbeitseinheit auseinandergebaut werden kann. Unter der Wendung "werkzeuglos trennbar" soll insbesondere verstanden werden, dass die Verbindung zwischen der Arbeitseinheit und der Bedieneinheit von einem Bediener händisch lösbar ausgebildet ist, das heißt, dass der Bediener zum Lösen der Verbindung kein Hilfsmittel benötigt.

Des Weiteren wird vorgeschlagen, dass die Verbindungsvorrichtung zumindest ein Rastelement und ein Federelement aufweist, das dazu vorgesehen ist, eine Kraft auf das Rastelement zu bewirken, wodurch konstruktiv einfach ein besonders komfortabler Auseinanderbau möglich ist. Unter einem "Rastelement" soll insbesondere ein Element verstanden werden, das dazu vorgesehen ist, bei einer formschlüssigen Verbindung eine verbindende Kraft auszuüben. Vorzugsweise ist das Rastelement zum Lösen der formschlüssigen Verbindung bewegbar gelagert. Insbesondere soll unter einem "Federelement" eine, dem Fachmann als sinnvoll erscheinende Torsions-, Biege-, Zugund/oder Gasfeder verstanden werden. Vorteilhaft ist das Federelement als eine Druckfeder ausgebildet. Besonders vorteilhaft ist das Federelement als eine Schraubenfeder ausgebildet.

Zudem wird vorgeschlagen, dass die Bedieneinheit eine Kulissenführung aufweist, die die Bedienelemente zumindest bei einer Arbeitsbewegung führt. Unter einer "Kulissenführung" soll insbesondere eine Führung verstanden werden, bei der eine Erhebung, insbesondere ein Stift, in einer Vertiefung, insbesondere in einem Kanal, zumindest von Seitenwänden der Vertiefung geführt, gelagert ist. Insbesondere soll unter dem Begriff "Arbeitsbewegung" eine Bewegung verstanden werden, die eine Betätigung des Arbeitsmittels verursacht. Durch die Kulissenführung sind eine besonders große Stabilität und eine konstruktiv besonders einfache und trennbare Verbindung zwischen der Bedieneinheit und der Arbeitseinheit möglich.

Des Weiteren wird vorgeschlagen, dass das chirurgische Instrument eine Verriegelungsvorrichtung umfasst, die dazu vorgesehen ist, eine Verriegelungskraft zwischen den Bedienelementen zu bewirken. Unter einer "Verriegelungsvorrichtung" soll insbesondere eine Vorrichtung verstanden werden, die in zumindest einem Betriebszustand eine Bewegung des Arbeitsmittels in zumindest eine Richtung verhindert. Vorzugsweise verhindert die Verriegelungsvorrichtung eine Bewegung der Bedienelemente relativ zueinander in zumindest eine Richtung. Durch die Verriegelungsvorrichtung ist ein besonders komfortables Arbeiten mit dem chirurgischen Instrument möglich. Vorzugsweise ist die Verriegelungsvorrichtung in einem Betriebszustand von den Bedienelementen formschlüssig befestigt angeordnet.

Ferner wird vorgeschlagen, dass die Verriegelungsvorrichtung werkzeuglos von den Bedienelementen zur Reinigung und/oder Desinfizierung trennbar befestigt ist, wodurch eine besonders effiziente und komfortable Reinigung und/oder Desinfizierung erreicht werden kann.

Zudem wird vorgeschlagen, dass die Verbindungsvorrichtung zumindest ein Entriegelbedienmittel aufweist, das zumindest teilweise auf einer den Bedienelementen abgewandten Seite der Bedieneinheit angeordnet ist, wodurch eine besonders komfortable Betätigung des Entriegelbedienmittels möglich ist. Insbesondere soll unter einem "Entriegelbedienmittel" ein Mittel verstanden werden, dessen Betätigung eine Trennung von der Arbeitseinheit und den Bedieneinheiten zumindest einleitet. Vorzugsweise löst eine Betätigung des Entriegelbedienmittels eine Verriegelung zwischen der Arbeitseinheit und den Bedieneinheiten. Unter einer "den Bedienelementen abgewandten Seite der Bedieneinheit" soll insbesondere eine Seite der Bedieneinheit verstanden werden, die, von zumindest einem Punkt der Bedienelemente gesehen, hinter einem Gelenk der Bedieneinheiten angeordnet ist. Vorzugsweise liegen die Bedieneinheit und zumindest eines der Bedienelemente auf einer Geraden, die in zumindest einem Betriebszustand zwischen der Bedieneinheit und diesem Bedienelement das Gelenk schneidet.

Weiterhin geht die Erfindung aus von einer Arbeitseinheit eines chirurgischen Instruments, insbesondere eines minimal-invasiven chirurgischen Instruments, mit einem Schaft und einem Schub- und Zugelement, die relativ zueinander bewegbar sind.

Es wird vorgeschlagen, dass die Arbeitseinheit des chirurgischen Instruments ein Verbindungselement aufweist, das dazu vorgesehen ist, den Schaft und das Schub- und Zugelement unverlierbar zueinander anzuordnen. Unter einem "Schaft" soll insbesondere ein Element der Arbeitseinheit verstanden werden, das zumindest einen Bereich und/oder ein Element eines Arbeitsmittels der Arbeitseinheit, insbesondere eine Gelenkachse, und einen Verbindungsbereich mechanisch fest miteinander verbindet. Vorteilhaft ist in einem betriebsbereiten Zustand der Verbindungsbereich mit einer Bedieneinheit direkt und fest verbunden. Insbesondere soll unter einem "Schub- und Zugelement" ein Element der Arbeitseinheit verstanden werden, das eine Arbeitsbewegung von der Bedieneinheit zu dem Arbeitsmittel überträgt. Vorzugsweise überträgt das Schub- und Zugelement die Arbeitsbewegung durch einen Schaftbereich. Unter dem Begriff "Verbindungselement" soll insbesondere ein Element verstanden werden, das dazu vorgesehen ist, zumindest eine Bewegung des Schub- und Zugelements relativ zu dem Schaft durch eine Gegenkraft zu verhindern. Insbesondere soll unter der Wendung "unverlierbar zueinander anzuordnen" verstanden werden, dass das Verbindungselement in einem von insbesondere der Bedieneinheit getrennten Zustand verhindert, dass der Schaft und das Schub- und Zugelement so zueinander ausrichtbar sind, dass eine formschlüssige Verbindung zwischen dem Schaft und dem Schub- und Zugelement lösbar ist. Somit ist das Verbindungselement ein Teil einer formschlüssigen Verbindung zwischen dem Schaft und dem Schub- und Zugelement. Vorzugsweise sind durch die unverlierbare Anordnung des Schafts und des Schub- und Zugelements auch alle anderen Teile der Arbeitseinheit unverlierbar angeordnet.

Durch die erfindungsgemäße Ausgestaltung der Arbeitseinheit des chirurgischen Instruments kann eine besonders gute Reinigbarkeit und/oder Desinfizierbarkeit bei einem hohen Komfort erreicht werden und ein Verlust einzelner Bauteile, insbesondere kleiner Bauteile des Arbeitsmittels, vermieden werden.

In einer vorteilhaften Ausbildung der Erfindung wird vorgeschlagen, dass das Verbindungselement zumindest teilweise von einer Feder gebildet ist. Alternativ und/oder zusätzlich könnte das Verbindungselement zumindest teilweise von einer Klapp- und/oder Scharniervorrichtung gebildet sein. Insbesondere soll unter einer "Feder" eine, dem Fachmann als sinnvoll erscheinende Torsions-, Biege-, Zug- und/oder Gasfeder verstanden werden. Vorteilhaft ist das Verbindungselement zumindest teilweise von einer Blattfeder gebildet. Durch die Feder kann vorteilhaft auf einer Seite des Verbindungselements auf eine bewegliche Verbindung zwischen dem Verbindungselement und dem Schaft und/oder dem Schub- und Zugelement verzichtet werden. Dadurch können konstruktiver Aufwand und Kosten gespart werden.

Des Weiteren wird vorgeschlagen, dass das Verbindungselement mit dem Schaft und/oder mit dem Schub- und Zugelement fest verbunden ist, wodurch geringe Kosten und ein geringer konstruktiver Aufwand erreicht werden können. Unter der Wendung "fest verbunden" soll insbesondere zumindest teilweise relativ zueinander unbeweglich verbunden verstanden werden. Vorteilhaft ist das Verbindungselement stoffschlüssig mit dem Schaft und/oder mit dem Schub- und Zugelement verbunden.

Ferner wird vorgeschlagen, dass der Schaft und/oder das Schub- und Zugelement eine zumindest teilweise T-förmige Nut aufweist, die in zumindest einem Betriebszustand das Verbindungselement zumindest teilweise führt. Unter einer "T-förmigen Nut" soll insbesondere eine Nut mit zwei Hinterschneidungen verstanden werden. Alternativ oder zusätzlich könnte der Schaft und/oder das Schub- und Zugelement eine L-förmige Nut und/oder eine andere, dem Fachmann als sinnvoll erscheine Nut aufweisen. Unter "führen" soll in diesem Zusammenhang insbesondere verstanden werden, dass die Nut dazu vorgesehen ist, auf das Verbindungselement in zumindest eine Richtung, vorteilhaft in alle Richtungen, senkrecht zu einer Haupterstreckungsrichtung der Nut, eine Kraft zu bewirken. Durch die T-förmige Nut können geringe Kosten und ein geringer konstruktiver Aufwand erreicht werden.

Zudem wird vorgeschlagen, dass das Schub- und Zugelement zumindest teilweise aus dem Schaft heraus schwenkbar gelagert ist, wodurch die Arbeitseinheit besonders vorteilhaft gereinigt und/oder desinfiziert werden kann. Unter der Wendung "aus dem Schaft heraus schwenkbar" soll insbesondere verstanden werden, dass das Schub- und Zugelement um eine Drehachse relativ zu dem Schaft in eine Position bewegbar ist, in der zumindest ein Bereich des Schub- und Zugelements in eine Richtung senkrecht zu einer Haupterstreckungsrichtung des Schafts um mehr als 2 mm, vorteilhaft mehr als 10 mm, von jedem Bereich des Schafts beabstandet angeordnet ist.

Weiterhin geht die Erfindung aus von einem Verfahren zur Montage eines chirurgischen Instruments, insbesondere eines minimal-invasiven chirurgische Instruments, mit einer Arbeitseinheit, einer Bedieneinheit und einer Verbindungsvorrichtung, die zumindest ein Rastelement und ein Entriegelbedienmittel aufweist, wobei das Rastelement dazu vorgesehen ist, die Arbeitseinheit mit der Bedieneinheit zu verrasten, wobei das Rastelement in die Bedieneinheit eingebracht und anschließend das Entriegelbedienmittel mit der Bedieneinheit fest verbunden wird. Insbesondere soll unter dem Begriff "Montage" ein Zusammenbau bei einer Herstellung des chirurgischen Instruments verstanden werden. Unter einer Montage soll nicht der Zusammenbau durch einen Bediener verstanden werden. Unter einem "Entriegelbedienmittel" soll insbesondere ein Mittel der Verbindungsvorrichtung verstanden werden, das dazu vorgesehen ist, von einem Bediener betätigt eine Verbindung zwischen der Arbeitseinheit und der Bedieneinheit zu lösen. Insbesondere soll unter dem Begriff "eingebracht" verstanden werden, dass das Rastelement in die Bedieneinheit eingeschoben wird und dabei in zumindest einem Betriebszustand von der Bedieneinheit in zwei parallele Richtungen verschiebbar gelagert ist. Vorteilhaft wird das Rastelement stoffschlüssig mit der Bedieneinheit verbunden. Alternativ oder zusätzlich könnten das Rastelement und die Bedieneinheit kraftschlüssig oder formschlüssig miteinander verbunden werden. Durch das erfindungsgemäße Verfahren kann konstruktiv einfach eine besonders stabile und komfortabel zu bedienende Verbindungsvorrichtung mit besonders effektiv reinigbaren und/oder desinfizierbaren Oberflächen erreicht werden. Des Weiteren wird vorgeschlagen, dass die Verbindungsvorrichtung ein Federelement und ein Verschlusselement aufweist, wobei das Federelement und das Verschlusselement in die Bedieneinheit eingebracht werden und das Verschlusselement mit der Bedieneinheit fest verbunden wird, wodurch besonders effektiv reinigbare und/oder desinfizierbare Oberflächen erreicht werden können. Unter einem "Verschlusselement" soll insbesondere ein Element verstanden werden, das dazu vorgesehen ist, eine Öffnung der Bedieneinheit zu verschließen. Vorzugsweise verschließt das Verschlusselement die Öffnung, durch die das Rastelement und das Federelement in die Bedieneinheit eingebracht werden. Vorteilhaft wird das Verschlusselement stoffschlüssig mit der Bedieneinheit verbunden. Alternativ könnten das Verschlusselement und die Bedieneinheit kraftschlüssig oder formschlüssig miteinander verbunden werden. Die Erfindung wird im Anspruch 1 definiert und weitere Ausführungsformen finden sich in den abhängigen Ansprüchen.

### Zeichnung

Weitere Vorteile ergeben sich aus der folgenden Zeichnungsbeschreibung. In der Zeichnung sind zwei Ausführungsbeispiele der Erfindung dargestellt. Die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: ein betriebsbereites chirurgisches Instrument mit einer Arbeitseinheit, einer Bedieneinheit und einer Verbindungsvorrichtung,
- Fig. 2: die Bedieneinheit des chirurgischen Instruments aus Figur 1 mit zwei voneinander getrennt angeordneten Bedienelementen,
- Fig. 3: eine Teilansicht der Arbeitseinheit des chirurgischen Instruments aus Figur 1,
- Fig. 4: eine perspektivische Teilansicht der Arbeitseinheit des chirurgischen Instruments aus Figur 1,
- Fig. 5: eine perspektivische Ansicht der Verbindungsvorrichtung des chirurgischen Instruments aus Figur 1,
- Fig. 6: die Arbeitseinheit und die Verbindungsvorrichtung des chirurgischen Instruments aus Figur 1 in einem verrasteten Betriebszustand,
- Fig. 7: eine perspektivische Teilansicht der Bedieneinheit und der Verbindungsvorrichtung des chirurgischen Instruments aus Figur 1,
- Fig. 8: eine perspektivische Ansicht einer Verbindungsvorrichtung des chirurgischen Instruments aus Figur 1,
- Fig. 9: ein alternatives Ausführungsbeispiel eines Entriegelbedienmittels eines chirurgischen Instruments aus Figur 1 und
- Fig. 10: einen Schnitt des alternatives Ausführungsbeispiels des Entriegelbedienmittels aus Figur 9.

### Beschreibung der Ausführungsbeispiele

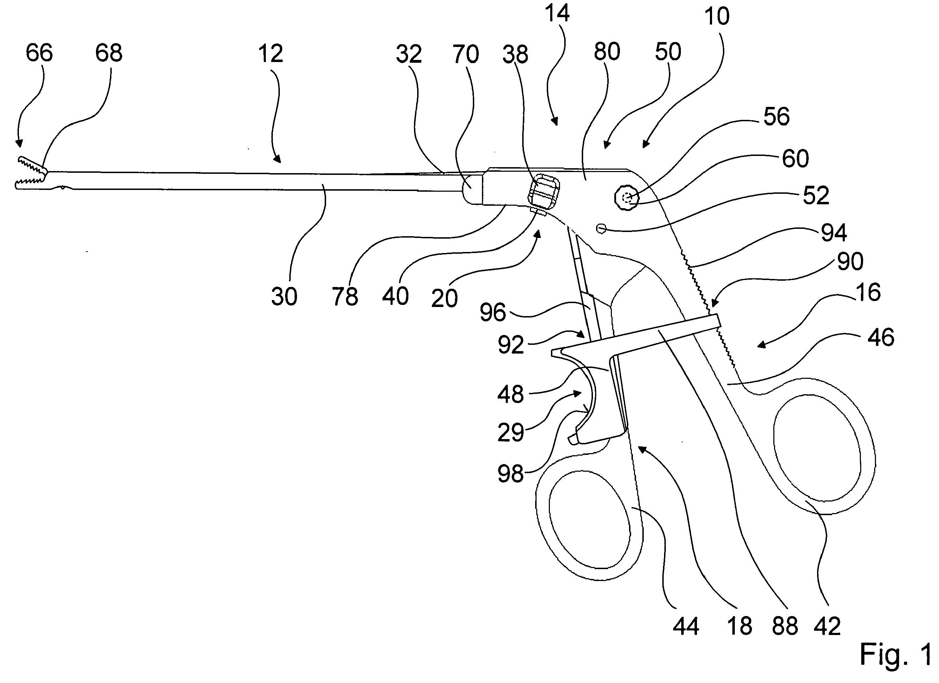

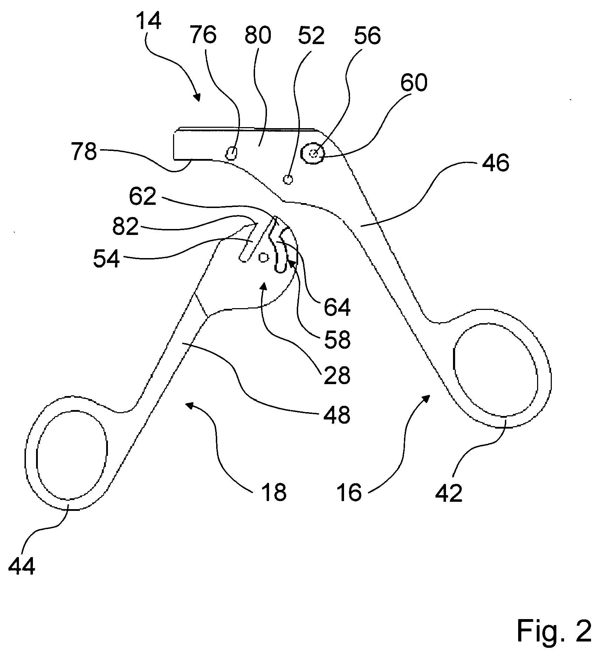

Figur 1 zeigt ein erfindungsgemäßes chirurgisches Instrument 10, das als minimal-invasives chirurgisches Instrument ausgebildet ist, mit einer Arbeitseinheit 12 und einer Bedieneinheit 14. Die Bedieneinheit 14 ist scherengriffartig ausgebildet. Wie Figur 2 zeigt, weist die Bedieneinheit 14 ein erstes Bedienelement 16 und ein zweites Bedienelement 18 auf, die zur Reinigung und/oder Desinfizierung voneinander trennbar verbunden sind. Die Bedienelemente 16, 18 weisen jeweils einen Griffbereich 42, 44 auf. Die Griffbereiche 42, 44 sind ringförmig ausgebildet. Dadurch kann ein nicht näher dargestellter Bediener in verschiedene Richtungen eine Kraft auf die Griffbereiche 42, 44 bewirken.

Die Griffbereiche 42, 44 sind jeweils über einen Steg 46, 48 der Bedienelemente 16, 18 mit einem Gelenk 50 verbunden. Die Stege weisen in einem betriebsbereiten Zustand jederzeit einen Winkel zu einer Haupterstreckungsrichtung der Arbeitseinheit 12 auf, der größer als 45 Grad ist. Das Gelenk 50 weist eine Kulissenführung 28 und eine Gelenkachse 52 auf. Die Gelenkachse 52 ist stoffschlüssig mit dem ersten Bedienelement 16 verbunden. Das zweite Bedienelement 18 weist eine Aussparung 54 auf, in die die Gelenkachse 52 bei einem Zusammenbau, das heißt insbesondere nach einer Reinigung und/oder Desinfizierung, von einem Bediener einführbar ist. Die Aussparung 54 erstreckt sich parallel zu dem Steg 48 des zweiten Bedienelements 18. In einem betriebsbereiten Zustand kommt die Gelenkachse 52 an einem inneren Ende der Aussparung 54 zu liegen.

Die Kulissenführung 28 weist einen Stift 56 und einen Kanal 58 auf. Der Stift 56 weist eine Basis 60 auf, die mit dem ersten Bedienelement 16 verschweißt ist. Der Stift 56 erstreckt sich in einen Gelenkbereich des Gelenks 50 hinein. Der Kanal 58 ist in das zweite Bedienelement 18 eingebracht. Er weist einen Einführungsbereich 62 und einen Arbeitsbereich 64 auf. Der Arbeitsbereich 64 erstreckt sich halbkreisförmig um das innere Ende der Aussparung 54. Der Einführungsbereich 62 erstreckt sich parallel zu der Aussparung 54.

Bei einer bestimmten, in Figur 2 dargestellten Ausrichtung der Bedienelemente 16, 18 zueinander, ist die Gelenkachse 52 und der Stift 56 in die Aussparung 54 und den Einführungsbereich 62 des Kanals 58 in eine Richtung parallel zu der Aussparung 54 einführbar. Nach dem Einführen sind die Bedienelemente 16, 18 zueinander um die Gelenkachse 52 schwenkbar. Dabei läuft der Stift 56 in dem Arbeitsbereich 64 des Kanals 58. Wenn der Stift 56 in dem Arbeitsbereich 64 angeordnet ist, verhindert diese Anordnung, dass die Bedienelemente 16, 18 in eine Richtung parallel zu der Aussparung 54, das heißt ohne vorheriges Schwenken, trennbar sind. Die Bedienelemente 16, 18 sind lediglich in der in Figur 2 dargestellten Ausrichtung trennbar.

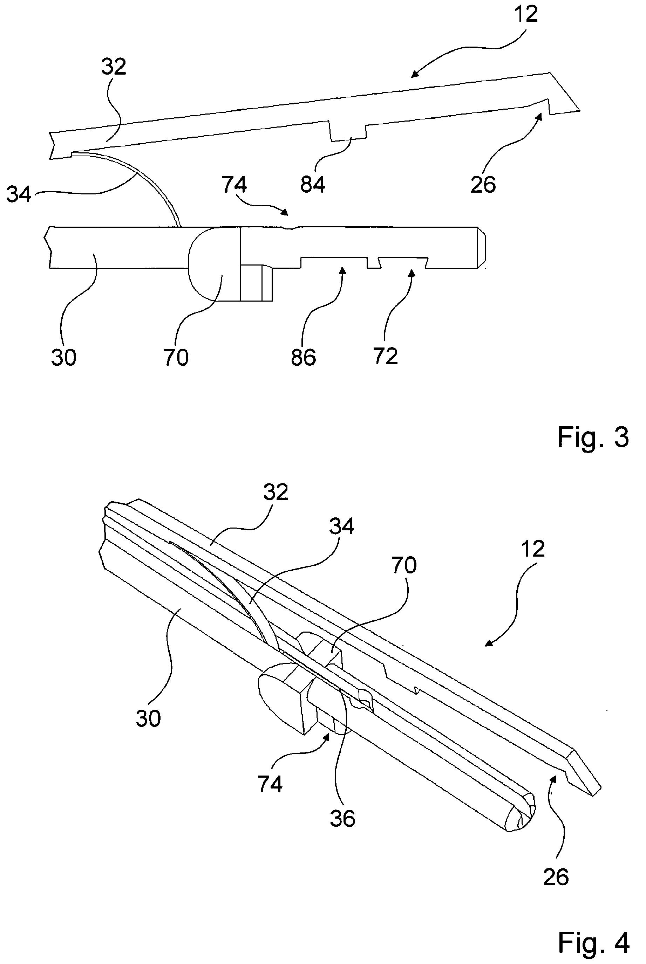

Die in den Figuren 3 und 4 näher dargestellte Arbeitseinheit 12 weist einen Schaft 30, ein Schub- und Zugelement 32 und ein Arbeitsmittel 66 auf. Der Schaft 30 ist in einem betriebsbereiten Zustand mechanisch fest mit der Bedieneinheit 14 verbunden, und zwar mit dem ersten Bedienelement 16 der Bedieneinheit 14. Das Arbeitsmittel 66 ist als ein Zangenmittel, genauer gesagt als ein krokodilmaulförmiges Arbeitsmittel, ausgebildet. Es ist teilweise einstückig mit dem Schaft 30 ausgebildet. Ein Teil 68 des Arbeitsmittels 66 ist relativ zu dem Schaft 30 und dem Schub- und Zugelement 32 bewegbar. Dieses Teil 68 ist wirkungsmäßig mit dem Schub- und Zugelement 32 verbunden.

Zum Verbinden mit der Bedieneinheit 14 weist der Schaft 30 einen Anschlag 70 und eine Rastaussparung 72 auf. Der Anschlag 70 ist als eine Verdickung des Schafts 30 senkrecht zu einer Haupterstreckungsrichtung des Schafts 30 ausgebildet. Der Anschlag 70 verhindert in einem betriebsbereiten Zustand eine Bewegung der Arbeitseinheit 12 in Richtung der Bedieneinheit 14. In die Rastaussparung 72 greift ein Rastelement 22 ein und verhindert in einem betriebsbereiten Zustand eine Bewegung der Arbeitseinheit 12 in eine Richtung von der Bedieneinheit 14 weg. Die Rastaussparung 72 ist in einem Verbindungsbereich 74 der Arbeitseinheit 12 angeordnet. Der Verbindungsbereich 74 ist dazu vorgesehen, in Haupterstreckungsrichtung des Schafts 30 in die Bedieneinheit 14 eingesteckt zu werden.

Zwischen dem Anschlag 70 und dem Arbeitsmittel 66 ist der Schaftbereich der Arbeitseinheit 12 angeordnet. Der Schaft 30 weist entlang seiner Haupterstreckungsrichtung eine Nut 36 auf. Die Nut 36 ist dazu vorgesehen, das Schub- und Zugelement 32 relativ zu dem Schaft 30 bewegbar zu lagern. Der Schaft 30 und das Schub- und Zugelement 32 sind mit dem Arbeitsmittel 66 wirkungsmäßig verbunden. Das Schub- und Zugelement 32 ist um eine am Arbeitsmittel 66 angeordnete Achse teilweise aus dem Schaft 30 heraus schwenkbar gelagert. Das Schub- und Zugelement 32 ist als eine Schub- und Zugstange ausgebildet.

Die Arbeitseinheit 12 weist ein Verbindungselement 34 auf. Das Verbindungselement 34 verbindet den Schaft 30 und das Schub- und Zugelement 32 auf einer dem Arbeitsmittel 66 abgewandten Seite des Schafts 30. Dabei verhindert das Verbindungselement 34, dass das Schub- und Zugelement 32 relativ zu dem Schaft 30 um mehr als 10 Grad schwenkbar ist. Dadurch verhindert das Verbindungselement 34 eine Bewegung des Schubund Zugelements 32 relativ zu dem Schaft 30, die eine formschlüssige Verbindung zwischen dem Schaft 30, dem Schub- und Zugelement 32 und dem bewegbaren Teil 68 des Arbeitsmittels 66 löst. Somit ordnet das Verbindungselement 34 den Schaft 30 und das Schub- und Zugelement 32 unverlierbar zueinander an. In einem betriebsbereiten Zustand ist das Verbindungselement 34 in der Nut 36 zwischen dem Schaft 30 und dem Schub- und Zugelement 32 angeordnet.

Das Verbindungselement 34 ist von einer Blattfeder gebildet und mit dem Schub- und Zugelement 32 verschweißt. Die Nut 36 des Schafts 30 ist in einem Bereich des Anschlags 70 T-förmig ausgebildet. Das Verbindungselement 34 weist zwei nicht näher dargestellte Flügel auf, die in die T-förmige Nut 36 eingreifen. Die Flügel führen das Verbindungselement 34 in Haupterstreckungsrichtung des Schafts 30 bewegbar. Das Verbindungselement 34 wird bei einer Montage zuerst in die T-förmige Nut 36 eingeführt und anschließend mit dem Schub- und Zugelement 32 verschweißt. Dabei ist das Verbindungselement 34 so angeordnet, dass es zum Ausführen aus der Nut 36 zu kurz ausgebildet ist.

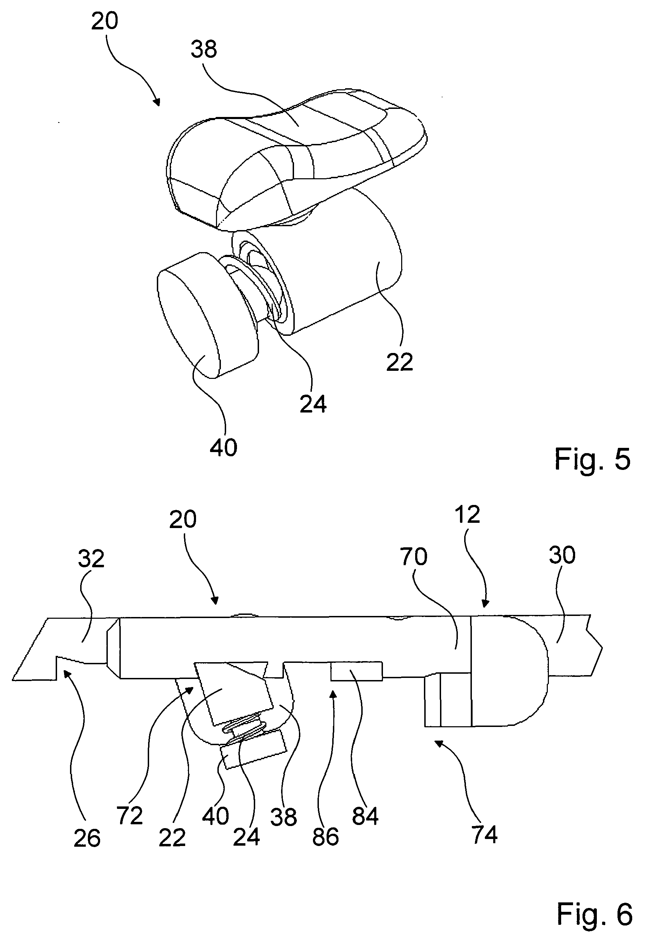

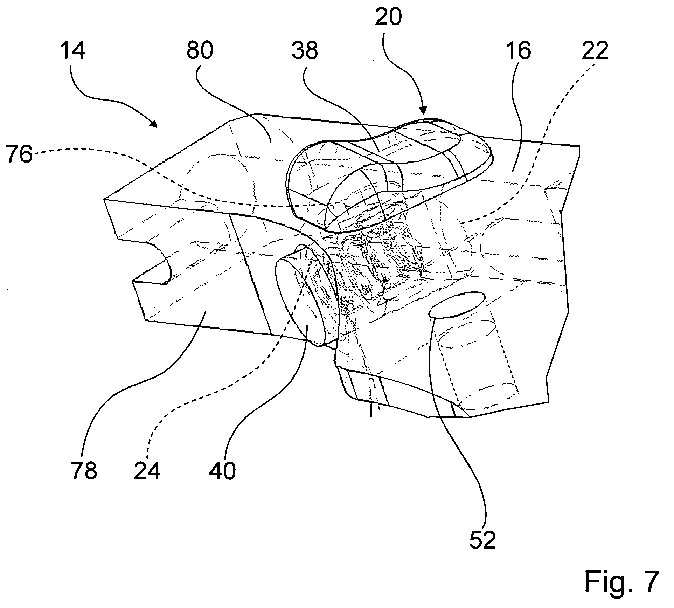

Die Bedieneinheit 14 weist eine in den Figuren 5, 6 und 7 abgebildete Verbindungsvorrichtung 20 auf. Die Verbindungsvorrichtung 20 weist das Rastelement 22 auf, das die Arbeitseinheit 12 und die Bedieneinheit 14 in einem betriebsbereiten Zustand werkzeuglos zur Reinigung und/oder Desinfizierung trennbar miteinander verrastet. Dazu weist die Verbindungsvorrichtung 20 ein Federelement 24, ein Entriegelbedienmittel 38 und ein Verschlusselement 40 auf. Das Federelement 24 ist als eine Druckfeder, und zwar als eine Schraubenfeder, ausgebildet. Es ist zwischen dem Rastelement 22 und dem Verschlusselement 40 angeordnet. Das Federelement 24 bewirkt eine Kraft auf das Rastelement 22, die das Rastelement 22 bei einem Einschieben der Arbeitseinheit 12 in die Bedieneinheit 16 mit der Arbeitseinheit 12 verrastet, und zwar mit der Aussparung 54 des Schafts 30. Dazu ist das Rastelement 22 in einer Bewegungsrichtung gelagert, die einen Winkel von 74 Grad zu der Haupterstreckungsrichtung des Schafts 30 aufweist. Dadurch ist ein selbsttätiges Lösen der Verbindung ausgeschlossen. Außerdem weist das Rastelement 22 eine Schrägfläche auf, die bei einem Einschieben der Arbeitseinheit 12 in die Bedieneinheit 14 das Rastelement 22 entrastet, das heißt zur Seite schiebt. Wenn die Arbeitseinheit 12 in einer betriebsbereiten Position relativ zur Bedieneinheit 14 ist, verrastet das Rastelement 22 selbsttätig.

Bei einer Montage der Arbeitseinheit 12 des chirurgischen Instruments 10 wird zuerst das Rastelement 22 in eine Aussparung des ersten Bedienelements 16 eingeführt. Die Aussparung ist als eine Bohrung ausgebildet. Des Weiteren ist die Aussparung auf einer Schmalseite 78 des ersten Bedienelements 16, dem zweiten Bedienelement 18 zugewandt, angeordnet. Anschließend wird das Entriegelbedienmittel 38 durch eine auf einer Breitseite 80 des ersten Bedienelements 16 angeordnete Aussparung 76 an das Rastelement 22 angelegt und mit dem Rastelement 22 mittels eines Lasers verschweißt. Danach werden das Federelement 24 und das Verschlusselement 40 in die Aussparung an der Schmalseite 78 eingebracht. Anschließend wird das Verschlusselement 40 mit dem ersten Bedienelement 16 verschweißt. Die Schweißstelle wird anschließend poliert.

Das Schub- und Zugelement 32 der Arbeitseinheit 12 weist einen Bewegungsanschlag 26 auf, der mit dem zweiten Bedienelement 18 in wirkungsmäßiger Verbindung steht. Dazu hakt der Bewegungsanschlag 26 an einer Kante 82 des zweiten Bedienelements 18 ein. Bei einer Bewegung des zweiten Bedienelements 18 bewegt die Kante 82 das Schub- und Zugelement 32 in Haupterstreckungsrichtung des Schub- und Zugelements 32. Das Schub- und Zugelement 32 weist einen Anschlag 84 auf, der in eine Aussparung 86 des Schafts 30 greift. Der Anschlag 84 beschränkt in einem betriebsbereiten Zustand einen Bewegungsspielraum des Schub- und Zugelements 32 relativ zu dem Schaft 30 in Haupterstreckungsrichtung des Schafts 30. Dabei verhindert der Anschlag 84 in einem betriebsbereiten Zustand eine Bewegung der Bedienelemente 16, 18 zueinander, die den Stift 56 in den Einführungsbereich 62 bewegt. Somit ordnet der Bewegungsanschlag 26 die Bedienelemente 16, 18 in einem betriebsbereiten Zustand unverlierbar zueinander an.

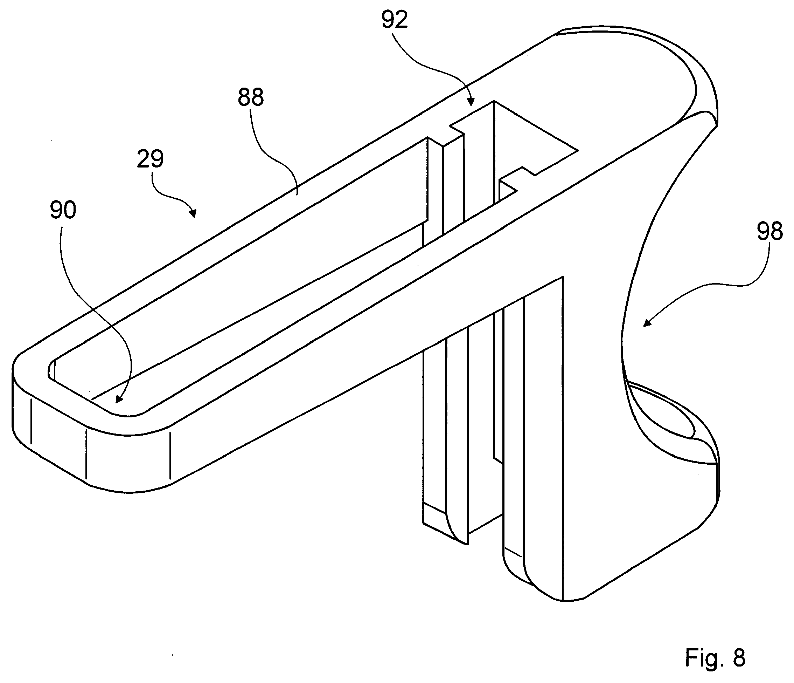

Wie in den Figuren 1 und 8 dargestellt, weist das chirurgische Instrument 10 eine Verriegelungsvorrichtung 29 auf. Die Verriegelungsvorrichtung 29 bewirkt in einem verriegelten Betriebszustand eine Verriegelungskraft zwischen den Bedienelementen 16, 18. Die Verriegelungsvorrichtung 29 verhindert eine Bewegung der Bedienelemente 16, 18 in eine Richtung, die einer Richtung entgegengesetzt ist, in die ein Bediener eine Kraft auf die Bedienelemente 16, 18 bewirkt. Die Verriegelungsvorrichtung 29 weist einen Ringbereich 88 auf, der in einem betriebsbereiten Zustand die Bedienelemente 16, 18 teilweise umschließt. Der Ringbereich 88 ist im Wesentlichen rechteckig ausgebildet. Zwei Innenflächen 90, 92 des Ringbereichs 88 bewirken die Verriegelungskraft. Die eine Innenfläche 90 rastet dazu in eine Zahnreihe 94 des ersten Bedienelements 16 ein. Die andere Innenfläche 92 umschließt eine T-förmige Nut 96 des zweiten Bedienelementes 18. Die Verriegelungsvorrichtung 29 ist auf dieser Nut 96 gleitend gelagert. Außerdem weist die Verriegelungsvorrichtung 29 einen Bedienbereich 98 auf. Der Bedienbereich 98 ist konkav, und zwar teilkreisförmig, ausgebildet. Der Bedienbereich 98 ist auf einer Seite des Ringbereichs 88 angeordnet, an dem die Innenfläche 90 angeordnet ist, die die T-förmige Nut 96 umschließt.

Die Verriegelungsvorrichtung 29 ist an den Bedienelementen 16, 18 zur Reinigung und/oder Desinfizierung werkzeuglos trennbar befestigt. Die Bedienelemente 16, 18 befestigen die Verriegelungsvorrichtung 29 zwischen den Griffbereichen 42, 44 und dem Gelenk 50 bzw. der Verbindungsvorrichtung 20 formschlüssig. Wenn das erste Bedienelement 16 von dem zweiten Bedienelement 18 getrennt ist, ist die Verriegelungsvorrichtung 29 vor den Bedienelementen 16, 18 trennbar.

Bei einem Zusammenbau des chirurgischen Instruments 10 wird zuerst das zweite Bedienelement 18 in das erste Bedienelement 18 in der in Figur 2 dargestellten Ausrichtung eingeführt. Anschließend wird das zweite Bedienelement 18 möglichst weit in Richtung des ersten Bedienelements 16, das heißt die Stege 46, 48 werden aufeinander zu geschwenkt. Danach wird die Arbeitseinheit 12 in das erste Bedienelement 16 eingesteckt. Dabei schwenkt das zweite Bedienelement 18 um etwa 45 Grad in Richtung der in Figur 2 dargestellten Ausrichtung. Um die in Figur 2 dargestellte Ausrichtung zu erreichen, müsste das zweite Bedienelement 18 um etwa 50 Grad geschwenkt werden. Dies verhindert jedoch der Bewegungsanschlag 26.

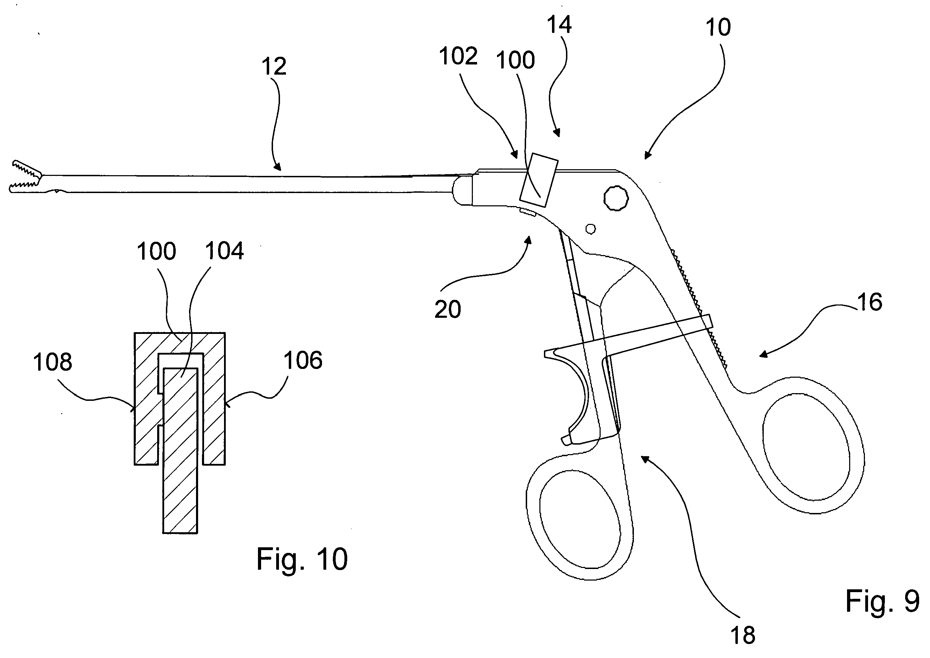

In den Figuren 9 und 10 ist ein weiteres Ausführungsbeispiel der Erfindung gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung des anderen Ausführungsbeispiels, insbesondere der Figuren 1 bis 8, verwiesen werden kann.

Figuren 9 und 10 zeigen ein weiteres Ausführungsbeispiel eines erfindungsgemäßen chirurgischen Instruments 10 mit einer Arbeitseinheit 12 und einer Bedieneinheit 14 und einer Verbindungsvorrichtung 20. Die Bedieneinheit 14 weist zwei zur Reinigung und/oder Desinfizierung voneinander trennbare Bedienelemente 16, 18 auf. Die Verbindungsvorrichtung 20 verbindet die Arbeitseinheit 12 und die Bedieneinheit 14 zur Reinigung und/oder Desinfizierung trennbar miteinander. Die Verbindungsvorrichtung 20 weist ein Entriegelbedienmittel 100 auf. Das Entriegelbedienmittel 100 ist teilweise auf einer den Bedienelementen 16, 18 abgewandten Seite 102 der Bedieneinheit 14 angeordnet. Das Entriegelbedienmittel 100 ist U-förmig ausgebildet. Es umschließt einen Teil 104 der Bedieneinheit 14. Somit ist das Entriegelbedienmittel 100 dazu vorgesehen, zur Betätigung mit einem Finger teilweise in Richtung des Teils 104 der Bedieneinheit 14 gedrückt zu werden und mit zwei Fingern auf zwei dem Teil 104 der Bedieneinheit 14 abgewandten, gegenüberliegenden Seiten gegriffen zu werden.

**Bezugszeichen**

| | | | |
|---|---|---|---|
| 10 | Chirurgisches Instrument | 58 | Kanal |
| | | 60 | Basis |
| 12 | Arbeitseinheit | 62 | Einführungsbereich |
| 14 | Bedieneinheit | 64 | Arbeitsbereich |
| 16 | Bedienelement | 66 | Arbeitsmittel |
| 18 | Bedienelement | 68 | Teil |
| 20 | Verbindungsvorrichtung | 70 | Anschlag |
| 22 | Rastelement | 72 | Rastaussparung |
| 24 | Federelement | 74 | Verbindungsbereich |
| 26 | Bewegungsanschlag | 76 | Aussparung |
| 28 | Kulissenführung | 78 | Schmalseite |
| 29 | Verriegelungsvorrichtung | 80 | Breitseite |
| | | 82 | Kante |
| 30 | Schaft | 84 | Anschlag |
| 32 | Schub- und Zugelement | 86 | Aussparung |
| 34 | Verbindungselement | 88 | Ringbereich |
| 36 | Nut | 90 | Innenfläche |
| 38 | Entriegelbedienmittel | 92 | Innenfläche |
| 40 | Verschlusselement | 94 | Zahnreihe |
| 42 | Griffbereich | 96 | Nut |
| 44 | Griffbereich | 98 | Bedienbereich |
| 46 | Steg | 100 | Entriegelbedienmittel |
| 48 | Steg | 102 | Seite |
| 50 | Gelenk | 104 | Teil |
| 52 | Gelenkachse | 106 | Seite |
| 54 | Aussparung | 108 | Seite |
| 56 | Stift | | |

## Patentansprüche

1. Chirurgisches Instrument, insbesondere minimal-invasives chirurgisches Instrument mit einer Arbeitseinheit (12) und einer Bedieneinheit (14), die zwei zur Reinigung und/oder Desinfizierung voneinander trennbare Bedienelemente (16, 18) aufweist, und einer Verbindungsvorrichtung (20), die dazu vorgesehen ist, die Arbeitseinheit (12) und die Bedieneinheit (14) zur Reinigung und/oder Desinfizierung trennbar miteinander zu verbinden, wobei die Arbeitseinheit (12) zumindest einen Bewegungsanschlag (26) aufweist, **dadurch gekennzeichnet, dass** der Bewegungsanschlag (26) dazu vorgesehen ist, die Bedienelemente (16, 18) unverlierbar zueinander anzuordnen, wobei der Bewegungsanschlag (26) in einem betriebsbereiten Zustand verhindert, dass die Bedienelemente (16, 18) so zueinander ausrichtbar sind, dass eine formschlüssige Verbindung zwischen den beiden Bedienelementen (16, 18) lösbar ist.

2. Chirurgisches Instrument nach Anspruch 1,
**dadurch gekennzeichnet, dass**
die Verbindungsvorrichtung (20) dazu vorgesehen ist, die Arbeitseinheit (12) und die Bedieneinheit (14) werkzeuglos trennbar zu verbinden.

3. Chirurgisches Instrument zumindest nach Anspruch 2,
**dadurch gekennzeichnet, dass**
die Verbindungsvorrichtung (20) zumindest ein Rastelement (22) und ein Federelement (24) aufweist, das dazu vorgesehen ist, eine Kraft auf das Rastelement (22) zu bewirken.

4. Chirurgisches Instrument nach einem der vorhergehenden Ansprüche,
**dadurch gekennzeichnet, dass**
die Bedieneinheit (14) eine Kulissenführung (28) aufweist, die die Bedienelemente (16, 18) zumindest bei einer Arbeitsbewegung führt.

5. Chirurgisches Instrument nach einem der vorhergehenden Ansprüche,
**gekennzeichnet durch**
eine Verriegelungsvorrichtung (29), die dazu vorgesehen ist, eine Verriegelungskraft zwischen den Bedienelementen (16, 18) zu bewirken.

6. Chirurgisches Instrument nach Anspruch 5,
**dadurch gekennzeichnet, dass**
die Verriegelungsvorrichtung (29) in zumindest einem Betriebszustand an den Bedienelementen (16, 18) zur Reinigung und/oder Desinfizierung werkzeuglos trennbar befestigt ist.

7. Chirurgisches Instrument nach einem der vorhergehenden Ansprüche,
**dadurch gekennzeichnet, dass**
die Verbindungsvorrichtung (20) zumindest ein Entriegelbedienmittel (100) aufweist, das zumindest teilweise auf einer den Bedienelementen (16, 18) abgewandten Seite (102) der Bedieneinheit (14) angeordnet ist.

8. Verfahren zur Montage eines chirurgischen Instruments (10) mit einer Arbeitseinheit (12), wobei die Arbeitseinheit zumindest einen Bewegungsanschlag (26) aufweist, der dazu vorgesehen ist, zwei Bedienelemente (16, 18) einer Bedieneinheit (14) unverlierbar zueinander anzuordnen, wobei der Bewegungsanschlag (26) in einem betriebsbereiten Zustand verhindert, dass die Bedienelemente (16, 18) so zueinander ausrichtbar sind, dass eine formschlüssige Verbindung zwischen den beiden Bedienelementen (16, 18)lösbar ist, wobei das chirurgische Instrument eine Verbindungsvorrichtung (20) aufweist,
die zumindest ein Rastelement (22) und ein Entriegelbedienmittel (38) aufweist, wobei das Rastelement (22) dazu vorgesehen ist, die Arbeitseinheit (12) mit der Bedieneinheit (14) zu verrasten, wobei das Rastelement (22) in die Bedieneinheit (14) eingebracht und anschließend das Entriegelbedienmittel (38) mit der Bedieneinheit (14) fest verbunden wird.

9. Verfahren zur Montage eines chirurgischen Instruments nach Anspruch 8,
**dadurch gekennzeichnet, dass**
die Verbindungsvorrichtung (20) ein Federelement (24) und ein Verschlusselement (40) aufweist, wobei das Federelement (24) und das Verschlusselement (40) in die Bedieneinheit (14) eingebracht werden und das Verschlusselement (40) mit der Bedieneinheit (14) fest verbunden wird.

## Claims

1. Surgical instrument, in particular minimally invasive surgical instrument, with a working unit (12) and with a control unit (14) comprising two control elements (16, 18) which are separable for cleaning and/or disinfection purposes, and with a connecting device (20) which is provided to connect the working unit (12) and the control unit (14) to each other separably for cleaning and/or disinfection purposes, the working unit (12) comprising at least one movement stop (26), **characterised in that** the movement stop (26) is provided to arrange the control elements (16, 18) captively with respect to each other, wherein the movement stop (26) prevents, in a state ready for operation, the control elements (16, 18) from being orientable to each other in such a way that a form-fit connection between the two control elements (16, 18) is releasable.

2. Surgical instrument as claimed in claim 1,
**characterised in that**
the connecting device (20) is provided to connect the working unit (12) and the control unit (14) in such a way that they are separable without a tool.

3. Surgical instrument at least as claimed in claim 2,
**characterised in that**
the connecting device (20) comprises at least one latch element (22) and a spring element (24) which is provided to exert a force onto the latch element (22).

4. Surgical instrument as claimed in any one of the preceding claims,
**characterised in that**
the control unit (14) comprises a slotted guide (28) which guides the control elements (16, 18) at least in a working movement.

5. Surgical instrument as claimed in any one of the preceding claims,
**characterised by**
a locking device (29) which is provided to exert a locking force between the control elements (16, 18).

6. Surgical instrument as claimed in claim 5,
**characterised in that**
the locking device (29) is in at least one operative state fixed to the control elements (16, 18) in such a way that it is separable without a tool for cleaning and/or disinfection purposes.

7. Surgical instrument as claimed in any one of the preceding claims,
**characterised in that**
the connecting device (20) comprises at least one unlocking control means (100) which is arranged at least partly on a side (102) of the control unit (14) that faces away from the control elements (16, 18).

8. Method for assembly of a surgical instrument (10) with a working unit (12), the working unit comprising at least one movement stop (26) which is provided to arrange two control elements (16, 18) of a control unit (14) captively with respect to each other, the movement stop (26 preventing, in a state ready for operation, the control elements (16, 18) from being orientable to each other in such a way that a form-fit connection between the two control elements (16, 18) is releasable,
wherein the surgical instrument (10) comprises a connecting device (20) comprising at least one latch element (22) and an unlocking control means (38), the latch element (22) being provided to latch the working unit (12) with the control unit (14), the latch element (22) being introduced into the control unit (14) and the unlocking control means (38) being subsequently fixedly connected to the control unit (14).

9. Method for assembly of a surgical instrument as claimed in claim 8,
**characterised in that**
the connecting device (20) comprises a spring element (24) and a closure element (40), wherein the spring element (24) and the closure element (40) are introduced into the control unit (14) and the closure element (40) is fixedly connected to the control unit (14).

## Revendications

1. Instrument chirurgical, notamment instrument chirurgical minimalement invasive, avec une unité travailleuse (12) et une unité de commande (14) comprenant deux éléments de commande (16, 18), lesquels sont séparables l'un de l'autre pour la nettoyage et/ou désinfection, et avec un dispositif de raccordement (20) prévu à raccorder l'unité travailleuse (12) et l'unité de commande (14) l'une à l'autre pour la nettoyage et/ou désinfection de telle façon qu'elles sont séparables, l'unité travailleuse (12) comportant au moins un arrêt de mouvement (26), **caractérisé en ce que** l'arrêt de mouvement (26) est prévu à disposer les éléments de commande (16, 18) imperdablement l'un en rapport à l'autre, l'arrêt de mouvement (26) empêchant, dans un état opérationnel, les éléments de commande (16, 18) d'être orientable l'un à l'autre de cette façon qu'un raccordement par forme entre les deux éléments de commande (16, 18) soit relâchable.

2. Instrument chirurgical selon la revendication 1,
**caractérisé en ce que**
le dispositif de raccordement (20) est prévue à raccorder l'unité travailleuse (12) et l'unité de commande (14) de telle façon qu'elles sont séparables sans d'outil.

3. Instrument chirurgical au moins selon la revendication 2,
**caractérisé en ce que**
le dispositif de raccordement (20) comporte au moins un élément d'encliquetage (22) et un élément de ressort (24), lequel est prévu à exercer une force sur l'élément d'encliquetage (22).

4. Instrument chirurgical selon l'une quelconque des revendications précédentes, **caractérisé en ce que**
l'unité de commande (14) comporte un guidage de coulisse (28), lequel guide les éléments de commande (16, 18) en au moins un mouvement de travail.

5. Instrument chirurgical selon l'une quelconque des revendications précédentes, **caractérisé par**
un dispositif de verrouillage (29) prévu à exercer une force de verrouillage entre les éléments de commande (16, 18).

6. Instrument chirurgical selon la revendication 5,
**caractérisé en ce que**
dans au moins un état opératif le dispositif de verrouillage (29) est fixé aux éléments de commande (16, 18) séparable sans d'outil pour la nettoyage et/ou désinfection.

7. Instrument chirurgical selon l'une quelconque des revendications précédentes, **caractérisé en ce que**
le dispositif de raccordement (20) comporte au moins un moyen de commande déverrouillant (100), lequel est disposé au moins partiellement à un côté (102) de l'unité de commande (14) détourné des éléments de commande (16, 18).

8. Procédé pour assemblage d'un instrument chirurgical (10) avec une unité travailleuse (12), l'unité travailleuse (12) comprenant au moins un arrêt de mouvement (26) prévu à disposer deux éléments de commande (16, 18) d'une unité de commande (14) imperdablement l'un par rapport à l'autre, l'arrêt de mouvement (26) empêchant, dans un état opérationnel, que les éléments de commande (16, 18) soient orientable l'un par rapport à l'autre de telle façon qu'un raccordement par forme entre les deux éléments de commande (16, 18) soit relâchable, l'instrument chirurgical comportant un dispositif de raccordement (20) comprenant au moins un élément d'encliquetage (22) et un moyen de commande déverrouillant (38), l'élément d'encliquetage (22) étant prévu à encliqueter l'unité travailleuse (12) avec l'unité de commande (14), l'élément d'encliquetage (22) étant introduit dans l'unité de commande (14) et ensuite le moyen de commande déverrouillant (38) étant fermement raccordé avec l'unité de commande (14).

9. Procédé pour assemblage d'un instrument chirurgical selon la revendication 8, **caractérisé en ce que**
le dispositif de raccordement (20) comporte un élément de ressort (24) et un élément de fermeture (40), l'élément de ressort (24) et l'élément de fermeture (40) étant introduits dans l'unité de commande (14) et l'élément de fermeture (40) étant fermement raccordé avec l'unité de commande (14).
